# EUROPÄISCHE PATENTSCHRIFT

(11) **EP 2 925 733 B1**
(45) Veröffentlichungstag und Bekanntmachung des Hinweises auf die Patenterteilung: **29.03.2017**
(21) Anmeldenummer: 13801496.4
(22) Anmeldetag: 22.11.2013
(51) Int. Cl.: C07D 249/14, C06B 25/00

(54) **3,3'-DINITRO-5,5'-BISTRIAZOL-1,1'-DIOL**
3,3'-DINITRO-5,5'-BISTRIAZOLE-1,1'-DIOL
3,3'-DINITRO-5,5'-BISTRIAZOL-1,1'-DIOL

(30) Priorität: 03.12.2012 DE 102012222086; 06.12.2012 DE 102012222424
(43) Veröffentlichungstag der Anmeldung: 07.10.2015
(73) Patentinhaber: Ludwig-Maximilians-Universität München, 80539 München (DE)
(72) Erfinder: KLAPÖTKE, Thomas M., 81477 München (DE); DIPPOLD, Alexander, 85560 Ebersberg (DE)
(74) Vertreter: Dr. Gassner & Partner mbB
(86) Internationale Anmeldenummer: PCT/EP2013/074500
(87) Internationale Veröffentlichungsnummer: WO 2014/086599

(56) Entgegenhaltungen:
- WO-A2-2005/035466
- DATABASE REGISTRY [Online] CHEMICAL ABSTRACTS SERVICE, COLUMBUS, OHIO, US; 11. Januar 2012 (2012-01-11), Chemical Library: XP002723640, Database accession no. 382159-83-3
- TSELINSKII I V ET AL: "Synthesis and study of thermal stability of 3-nitro-1,2,4-triazole N-nitroxy-and N-azidomethyl derivatives", RUSSIAN CHEMICAL BULLETIN, KLUWER ACADEMIC PUBLISHERS-PLENUM PUBLISHERS, NE, Bd. 58, Nr. 11, 25. September 2010 (2010-09-25), Seiten 2356-2361, XP019829769, ISSN: 1573-9171
- ALEXANDER A. DIPPOLD ET AL: "A Study of Dinitro-bis-1,2,4-triazole-1,1'-diol and Derivatives: Design of High-Performance Insensitive Energetic Materials by the Introduction of N-Oxides", JOURNAL OF THE AMERICAN CHEMICAL SOCIETY., Bd. 135, 2013, Seiten 9931-9937, XP002723641, USAMERICAN CHEMICAL SOCIETY, WASHINGTON, DC. ISSN: 0002-7863

## Beschreibung

Die Erfindung betrifft die neue Verbindung 3,3'-Dinitro-5,5'-Bistriazol-1,1'-diol, ein Salz davon, eine energetische Wirkmasse mit einem Salz davon und einer Verwendung mit einem Salz davon. Weiterhin betrifft die Erfindung ein Verfahren zur Herstellung von 3,3'-Dinitro-5,5'-Bistriazol-1,1'-diol sowie ein Verfahren zur Herstellung bestimmter Salze davon.

Es ist bekannt, Nitramine, wie beispielsweise Hexogen (RDX), Octogen (HMX) oder Hexanitroisowurtzitan (CL-20), als Sekundärsprengstoffe zu verwenden. Ein Nachteil dieser Nitramine und ihrer Reaktionsprodukte nach einer Detonation besteht in deren Toxizität und Umweltschädlichkeit. Es besteht jedoch ein Bedürfnis nach leistungsfähigeren Sekundärsprengstoffen. Diese sind zwar bereits bekannt, beispielsweise in Form von Dinitroazofuroxan oder Octanitrocuban. Ein Nachteil dieser Stoffe besteht jedoch in ihrer für Sekundärsprengstoffe hohen Sensitivität und deren sehr aufwändiger und zehn oder mehr Reaktionsschritte erfordernder Synthese. Weiterhin besteht ein Bedürfnis nach thermisch hoch stabilen Explosivstoffen mit einer höheren energetischen Leistung und einer höheren thermischen Stabilität als das üblicherweise verwendete Hexanitrostilben (HNS).

WO 2005/035466 A2 beschreibt Salze von Dinitrobistriazol als Treibmittel.

Aufgabe der vorliegenden Erfindung ist es, eine alternative energetische Wirkmasse bereitzustellen, welche einfach herzustellen ist und eine hohe Leistungsfähigkeit bei sicherer Handhabbarkeit und hoher thermischer Stabilität sowie vertretbarer Umweltschädlichkeit aufweist. Weiterhin sollen mögliche Bestandteile dieser energetischen Wirkmasse sowie ein Ausgangsstoff zur Herstellung dieser Bestandteile angegeben werden. Darüber hinaus soll eine Verwendung eines der Bestandteile, ein Verfahren zur Herstellung des Ausgangsstoffs sowie ein Verfahren zur Herstellung eines der Bestandteile der energetischen Wirkmasse angegeben werden.

Die Aufgabe wird durch die Merkmale der Ansprüche 1, 2, 4, 5, 6 und 10 gelöst. Zweckmäßige Ausgestaltungen ergeben sich aus den Merkmalen der Ansprüche 3 und 7 bis 9.

Erfindungsgemäß sind 3,3'-Dinitro-5,5'-Bistriazol-1,1'-diol sowie ein Salz davon, insbesondere ein Dihydroxylammoniumsalz, Diguanidiniumsalz oder Ditriaminoguanidiniumsalz davon vorgesehen. Weiterhin ist eine energetische Wirkmasse umfassend ein Dihydroxylammoniumsalz, Diguanidiniumsalz oder Ditriaminoguanidiniumsalz von 3,3'-Dinitro-5,5'-Bistriazol-1,1'-diol vorgesehen. Unter einer energetischen Wirkmasse wird hier eine nach deren Zündung deflagratativ oder detonativ reagierende Wirkmasse verstanden. Es kann sich dabei um eine pyrotechnische Wirkmasse handeln. Das Dihydroxylammoniumsalz von 3,3'-Dinitro-5,5'-Bistriazol-1,1'-diol (MAD-X1) zeigt eine Detonationsgeschwindigkeit im Bereich der Detonationsgeschwindigkeit von CL-20, die etwa um 500 ms⁻¹ höher ist als diejenige von RDX. MAD-X1 erfüllt damit die für einen Hochleistungssprengstoff geforderten Voraussetzungen. Die Berechnung der Detonationsgeschwindigkeiten erfolgte mit dem Programm EXPLO5, Version 5.05 (M. Sućeska, EXPLO5.04 program, Zagreb, Croatia, 2011; M. Sućeska, Calculation of detonation parameters by EXPLO5 computer program, Materials Science Forum, 2004, 465-466, 325-330; M. Sućeska, Calculation of the detonation properties of C-H-N-O explosives, Propellants, Explos., Pyrotech. 1991, 16, 197-202; M. Suceska, Evaluation of detonation energy from EXPLO5 computer code results, Propellants, Explos., Pyrotech. 1999, 24, 280-285; M. L. Hobbs, M. R. Baer, Proc. of the 10th Symp. (International) on Detonation, ONR 33395-12, Boston, MA, July 12-16, 1993, p. 409).

Weiterhin ist die Sensitivität von MAD-X1 herausragend. Während RDX beim Test auf Schlagempfindlichkeit mit der Fallhammermethode bereits bei 7,5 J reagiert und zur Verwendung durch den Zusatz von Bindemitteln und Weichmachern desensitiviert werden muss ist MAD-X1 bereits vollständig insensitiv gegenüber Schlag und Reibung. Mit der Schlaghammermethode wurde erst bei über 40 J eine Reaktion beobachtet und bei der mit einem Reibapparat gemessenen Reibempfindlichkeit waren 360 N erforderlich, um eine Reaktion auszulösen. Durch die Insensitivität gegenüber äußeren Stimuli ist die Sicherheit beim Herstellen und Handhaben gegenüber anderen energetischen Wirkmassen deutlich erhöht. MAD-X1 ist daher zur Herstellung insensitiver Munition sehr gut geeignet. Weiterhin ist die Dichte von MAD-X1 deutlich höher als diejenige von RDX. Weiterhin kann eine MAD-X1 enthaltende Wirkmasse ohne für eine Desensitivierung erforderliche Zusatzstoffe auskommen und auch wegen der geringen Dichte dieser Zusatzstoffe eine deutlich höhere Dichte aufweisen als eine RDX enthaltende Wirkmasse. Dies bedeutet, dass in einem gegeben Volumen eine höhere Masse einer MAD-X1 enthaltenden Wirkmasse untergebracht und damit eine höhere Leistung erreicht werden kann, als mit RDX.

Das Diguanidiniumsalz von 3,3'-Dinitro-5,5'-Bistriazol-1,1'-diol (MAD-X2) erfüllt nicht die für einen Hochleistungssprengstoff geforderten Voraussetzungen, weist aber dafür herausragende Eigenschaften im Hinblick auf thermische Stabilität und Insensitivität gegenüber äußeren Stimuli auf. Es zersetzt sich bei einer Heizrate von 5°C/min erst bei einer Temperatur von 341°C und übersteigt damit bei weitem die Zersetzungstemperatur von RDX und sogar die Zersetzungstemperatur von HNS. Auch die Detonationsgeschwindigkeit von 8242 cm⁻¹ ist höher als diejenige des üblicherweise verwendeten hitzeresistenten Sprengstoffs HNS.

Das Triaminoguanidiniumsalz von 3,3'-Dinitro-5,5'-Bistriazol-1,1'-diol (MAD-X3) verbindet eine hohe Detonationsgeschwindigkeit, die im Bereich der Detonationsgeschwindigkeit von RDX liegt mit einem sehr hohen Stickstoffgehalt von 60.07%. Der hohe Stickstoffgehalt führt beim Abbrand bzw. bei Detonation zur Entstehung eines verhältnismäßig großen Gasvolumens. In Kombination mit der hohen Detonationsgeschwindigkeit kann dadurch ein sehr starker Impuls bereitgestellt werden. Dadurch ist MAD-X3 besonders gut als Treibmittel geeignet. Weiterhin verringert ein hohes Stickstoff zu CO₂-Verhältnis eine Korrosion an Eisen enthaltenden Abschußrohren, wenn MAD-X3 als Treibmittel für ein Geschoß eingesetzt wird, weil dadurch die Entstehung von Eisencarbid reduziert wird. Zusätzlich zeigt MAD-X3 moderate Sensitivitäten gegenüber Reibung (360 N) und Schlag (10 J). MAD-X1, MAD-X2 und MAD-X3 können jeweils als Sprengstoff, als Treibmittel, als pyrotechnisches Gaserzeugungsmittel, z. B. in einem Gasgenerator, oder als Additiv zu einem Feststofftreibstoff verwendet werden.

Die Eigenschaften von MAD-X1, MAD-X2 und MAD-X3 im Vergleich zu Nitropenta (PETN), RDX, β-HMX, ε-CL-20 und HNS sind in der folgenden Tabelle dargestellt:

| | **PETN** | **RDX** | **β-HMX** | **ε-CL20** | **HNS** | **MAD-X1** | **MAD-X2** | **MAD-X3** |
|---|---|---|---|---|---|---|---|---|
| Formel | C₅H₈N₄O₁₂ | C₃H₆N₆O₆ | C₄H₈N₈O₈ | C₆H₆N₁₂O₁₂ | C₁₄H₆N₆O₁₂ | C₄H₈N₁₀O₈ | C₆H₁₂N₁₄O₆ | C₆H₁₈N₂₀O₆ |
| Molekulargewicht | 316,1 | 222,1 | 296,2 | 438,2 | 450,1 | 324,2 | 376,3 | 466,3 |
| IS [J]^{a} | 4 | 7,5 | 7 | 4 | 5 | >40 | >40 | 10 |
| FS [N]^{b} | 80 | 120 | 112 | 48 | 240 | 360 | 360 | 360 |
| ESD-Test [J]^{c} | 0,1 | 0,2 | 0,2 | - | - | 0,5 | 0,8 | 0,2 |
| *N* [%]^{d} | 17,72 | 37,8 | 37,8 | 38,3 | 18,7 | 43,21 | 52,12 | 60,07 |
| *Ω* [%]^{e} | -10,12 | -21,6 | -21,6 | -11,0 | -67,6 | -19,7 | -51,0 | -51,5 |
| T_{dec.} [°C]^{f} | 150 | 210 | 285 | 195 | 320 | 222 | 341 | 220 |
| Dichte [g cm⁻³]^{g} | 1,778 | 1,820 | 1,905 | 2,038 | 1,74 | 1,952 | 1,788 | 1,75 |
| Δ_{f}*U*° / kJ kg^{-1 h} | -1611 | 417 | 353 | 982 | 240 | 756 | 366 | 1858 |
| *-*Δ*_{E}U°* [kJ kg⁻¹]ⁱ | 6190 | 6125 | 6063 | 6473 | 5474 | 5980 | 4163 | 5105 |
| *T_{E}* [K]^{j} | 4306 | 4236 | 4117 | 4654 | 3982 | 4132 | 3038 | 3393 |
| p_{C-J} [kbar]^{k} | 320 | 348 | 392 | 446 | 242 | 413 | 276 | 316 |
| D [m s⁻¹]^{l} | 8320 | 8748 | 9058 | 9342 | 7446 | 9253 | 8242 | 8802 |
| Gasvolumen [l kg⁻¹]^{m} | 688 | 739 | 734 | 669 | 530 | 740 | 770 | 817 |

| | | | | | | | | |
|---|---|---|---|---|---|---|---|---|
| ^{a} Schlagempfindlichkeit (gemessen mittels der Fallhammermethode gemäß der Bundesanstalt für Materialforschung und -prüfung); ^{b} Reibempfindlichkeit (gemessen mit einem Reibapparat gemäß der Bundesanstalt für Materialforschung und -prüfung); ^{c} gemessen mit der elektrostatischen Entladungsvorrichtung der Firma OZM Research s.r.o., Tschechien; ^{d} Stickstoffgehalt; ^{e} Sauerstoffbilanz; ^{f} Zersetzungstemperatur gemäß DSC (Differential Scanning Calorimetry)-Messung (5 °C pro Minute); ^{g} ermittelt mittels Röntgendiffraktometrie bei etwa 100 K; ^{h} Bildungsenergie berechnet mittels der CBS-4M-Methode; ⁱ Explosionsenergie; ^{j} Explosionstemperatur; ^{k} Detonationsdruck; ^{l} Detonationsgeschwindigkeit; ^{m} ermittelt unter der Annahme ausschließlich gasförmiger Reaktionsprodukte. | | | | | | | | |

Ein weiterer Vorteil besteht in der Synthese von MAD-X1, MAD-X2 und MAD-X3, die einfach unter Verwendung günstiger Ausgangsmaterialien und einer geringen Zahl von Syntheseschritten erfolgen kann. Der letzte Syntheseschritt kann dabei jeweils in Ethanol erfolgen, woraus die darin unlöslichen Verbindungen MAD-X1, MAD-X2 und MAD-X3 als amorphes Pulver präzipitieren. Dies ist für die Formulierung einer energetischen Wirkmasse vorteilhaft, da durch das niedrige Oberfläche zu Volumen Verhältnis bei den kugelförmigen Partikeln des Pulvers gegenüber bei anderen Sprengstoffsynthesen häufig gebildeten Nadeln zur Herstellung der Wirkmasse weniger oder kein Weichmacher und weniger Bindemittel benötigt wird. Dadurch kann ein höherer Gehalt an Explosivstoff in der Wirkmasse und damit eine höhere Leistung der Wirkmasse erreicht werden. Weiterhin weisen die Verbindungen MAD-X1, MAD-X2 und MAD-X3 eine geringe Wasserlöslichkeit auf. Dies ist für die Weiterverarbeitung der Salze vorteilhaft.

Bei einem Test der Leistungsfähigkeit mittels des sogenannten SSSRTs (Small Scale Shock Reactivity Test), bei dem untersucht wird, wie weit ein Aluminium-Block durch einen zu untersuchenden Sprengstoff bei dessen Detonation ausgebeult wird, zeigte es sich, dass MAD-X1 nach dessen Zündung eine ähnliche Leistungsfähigkeit wie β-HMX und eine höhere Leistungsfähigkeit als RDX aufweist.

Da bekannt ist, dass beim SSSRT weniger sensitive Explosivstoffe eine größere Menge benötigen, um ihre Leistungsfähigkeit zu zeigen und sensitivere Explosivstoffe mit geringerer Leistungsfähigkeit eine scheinbar höhere Leistungsfähigkeit zeigen, kann davon ausgegangen werden, dass die tatsächlichen Leistungsfähigkeiten von MAD-X1 die Leistungsfähigkeit von β-HMX übersteigt.

Die Erfindung betrifft weiterhin die Verwendung eines Dihydroxylammoniumsalzes, Diguanidiniumsalzes oder Ditriaminoguanidiniumsalzes von 3,3'-Dinitro-5,5'-Bistriazol-1,1'-diol als Sprengstoff, Treibmittel, pyrotechnisches Gaserzeugungsmittel oder Additiv zu einem Feststofftreibstoff sowie ein Verfahren zur Herstellung von 3,3'-Dinitro-5,5'-Bistriazol-1,1'-diol, wobei 3,3'-Dinitro-5,5'-bis(1H-1,2,4-triazol) (DNBT) zum 3,3'-Dinitro-5,5'-bis(1,2,4-triazol)-1,1'-diol (DNBTO) oxidiert wird.

Das DNBT kann mittels 2KHSO₅·KHSO₄·K₂SO₄ oder einer anderen anorganischen oder organischen Peroxosäure, Perborat, Wasserstoffperoxid oder hypofluoriger Säure oder einem anderen Sauerstoffüberträger oxidiert werden. Die Oxidation kann in wässriger Lösung erfolgen. 2KHSO₅·KHSO₄·K₂SO₄ wird unter dem Handelsnamen Oxone^{®} von der Firma DuPont vertrieben. Der Zusatz von Oxone^{®} oder der anderen anorganischen oder organischen Peroxosäure, Perborat, Wasserstoffperoxid oder hypofluoriger Säure oder dem anderen Sauerstoffüberträger erfolgt vorzugsweise im Überschuss, um eine vollständige Oxidation sicherzustellen. Die Oxidation mit Oxone^{®} ist anderen Verfahren im Hinblick auf Kosten und Handhabung überlegen. Bei der Oxidation von DNBT kann das Verhältnis von Oxone^{®} und DNBT und/oder die Reaktionszeit variiert werden. Das DNBT kann durch Diazotierung von 3,3'-Diamino-5,5'-bis(1H-1,2,4-triazol) (DABT) in Schwefelsäure in Gegenwart von Nitrit erhalten werden.

Das DABT kann durch Reaktion von Oxalsäure und Aminoguanidinbicarbonat in Säure, insbesondere konzentrierter Salzsäure, Absondern des dabei gebildeten Zwischenprodukts und Erhitzen des Zwischenprodukts in einem basischen Medium erhalten werden.

Die Erfindung betrifft weiterhin ein Verfahren zur Herstellung von Dihydroxylammoniumsalz, Diguanidiniumsalz oder Ditriaminoguanidiniumsalz von 3,3'-Dinitro-5,5'-Bistriazol-1,1'-diol mit folgenden Schritten:
a) Inkubation von DNBTO mit Hydroxylamin, Hydroxylammoniumionen, Guanidiniumcarbonat, Guanidiniumionen, Guanidin oder Triaminoguanidin in alkoholischer Lösung und
b) Absondern des dabei erhaltenen Präzipitats.

Nachfolgend wird anhand von Ausführungsbeispielen die Synthese von MAD-X1, MAD-X2 und MAD-X3 schematisch dargestellt. Es zeigen:
- Fig. 1: eine schematische Darstellung der Synthese von DABT,
- Fig. 2: eine schematische Darstellung der Synthese von DNBT,
- Fig. 3: eine schematische Darstellung der Synthese von DNBTO,
- Fig. 4: eine schematische Darstellung der Synthese von MAD-X1,
- Fig. 5: eine schematische Darstellung der Synthese von MAD-X2 und
- Fig. 6: eine schematische Darstellung der Synthese von MAD-X3.

### 1. Synthese von DABT:

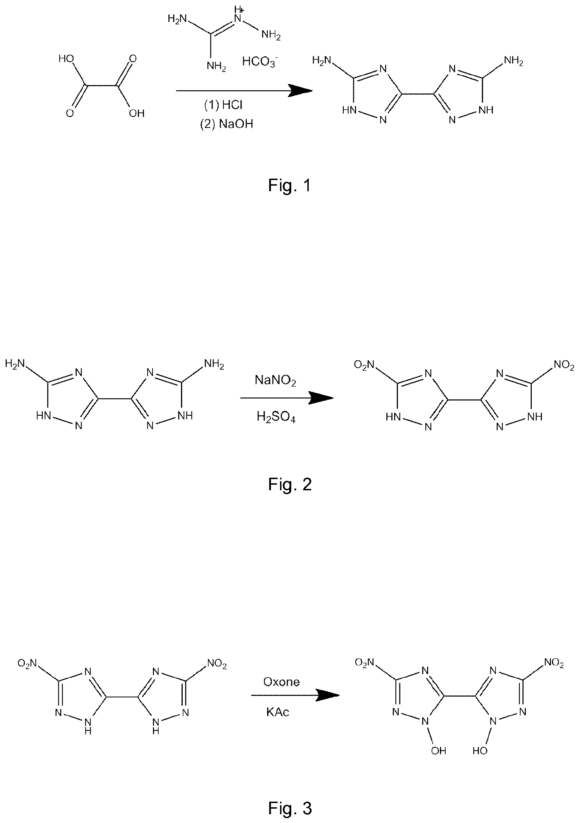

100 ml konzentrierte Salzsäure werden unter Rühren einer wässrigen Lösung von 20 g (159 mmol) Oxalsäure und 45,4 g (332 mmol) Aminoguanidinbicarbonat zugesetzt. Die Lösung wird bei 70°C für eine Stunde gerührt und dann auf Raumtemperatur abkühlen gelassen. Das dabei gebildete Präzipitat wurde durch Filtration als weißer Feststoff abgesondert. Der abgesonderte Feststoff wurde in 240 ml Wasser gelöst und mittels Natriumhydroxid auf einen pH-Wert von 14 eingestellt. Die Mischung wurde anschließend für eine Stunde unter Rückfluss gekocht, dann auf Raumtemperatur abgekühlt und mit konzentrierter Essigsäure auf einen pH-Wert von 4 angesäuert. Ein dadurch gebildetes Präzipitat wurde durch Filtration abgesondert. Dabei wurden 18,6 g (112 mmol) DABT als farbloser Feststoff erhalten. Dies entspricht einer Ausbeute von 70%. Die Synthese ist schematisch in Fig. 1 dargestellt.

### 2. Synthese von DNBT:

Eine Suspension von 11,9 g (72 mmol) DABT in 140 ml 20%iger Schwefelsäure wurde tropfenweise zu einer Lösung von 98,8 g (1,4 mol) Natriumnitrit (entspricht 20 eq.) in 140 ml Wasser bei 50°C zugesetzt. Danach wurde die Mischung für eine Stunde bei 50°C gerührt. Anschließend wurde das Reaktionsgemisch mit 20%iger Schwefelsäure solange angesäuert bis keine Entstehung von Stickstoffdioxid mehr beobachtet werden konnte. Dann wurde die Lösung auf 0°C abgekühlt. Das dabei gebildete Präzipitat wurde durch Filtration abgesondert, in kochendem Wasser gelöst, heiß filtriert um unlösliche Verunreinigungen abzusondern und die resultierende klare Lösung bei 5°C über Nacht gelagert. Dabei hat sich ein blassgrünes Präzipitat von DNBT-Dihydrat als kristalliner Feststoff gebildet. Der Feststoff wurde abgesondert. Die Ausbeute betrug 15,5 g, entsprechend 59 mmol oder 82%. Die entsprechende Diazotierungsreaktion ist in Fig. 2 dargestellt.

### 3. Synthese von DNBTO:

5 g (19 mmol) DNBT×2H₂O wurden in 300 ml einer mit 74,7 g (760 mmol) Kaliumacetat gepufferten wässrigen Lösung gelöst. Dazu wurden 117 g (0,38 mol) Oxone^{®} portionsweise zugesetzt. Die Mischung wurde anschließend für 60 Stunden bei 40°C gerührt. Danach wurde die Lösung mittels 150 ml Schwefelsäure (50 Gew.-%) angesäuert und mit 4 x 100 ml Ethylacetat extrahiert. Die vereinigten organischen Phasen wurden über Magnesiumsulfat getrocknet. Das Lösungsmittel wurde anschließend im Vakuum evaporiert. Der Rückstand wurde in 50 ml Benzol suspendiert und zur vollständigen Trocknung evaporiert. Dabei blieben 4 g (16 mmol) DNBTO als farbloser Feststoff zurück. Dies entspricht einer Ausbeute von 81 %. Die Reaktion ist schematisch in Fig. 3 dargestellt.

### 4. Synthese von MAD-X1:

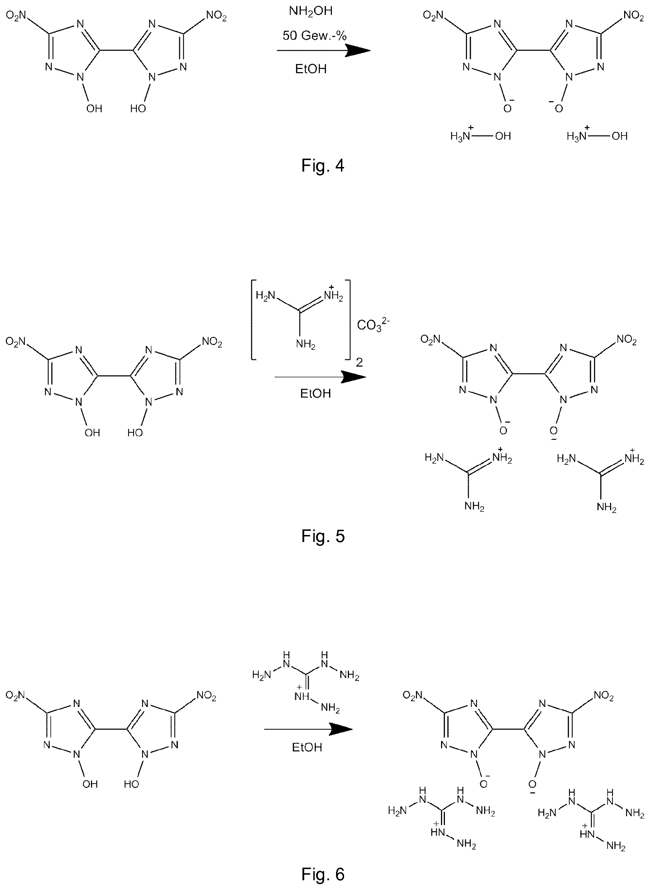

0,5 g (1,9 mmol) DNBTO wurden in 50 ml Ethanol gelöst. Der Lösung wurden 0,2 ml einer Lösung von Hydroxylamin in Wasser (50 Gew.-%, entspricht 2,2 eq.) zugesetzt. Die Mischung wurde für 10 min gerührt und das gebildete Präzipitat als oranger Feststoff durch Filtration abgesondert. Der abgesonderte Feststoff wurde in Luft getrocknet. Dabei wurden 0,6 g (1,9 mmol) MAD-X1 erhalten. Dies entspricht einer Ausbeute von 95%. Die Reaktion ist schematisch in Fig. 4 dargestellt.

### 5. Synthese von MAD-X2:

0,3 g (1,2 mmol) DNBTO wurden in 50 ml Ethanol gelöst. Der Lösung wurden 0,22 g (1,2 mmol) Guanidiniumcarbonat zugesetzt. Die Mischung wurde für 30 min auf 60°C erhitzt. Das dabei gebildete Präzipitat wurde als oranger Feststoff durch Filtration abgesondert. Der abgesonderte Feststoff wurde aus Wasser rekristallisiert. Dabei wurden 0,33 g (0,84 mmol) MAD-X2 erhalten. Dies entspricht einer Ausbeute von 71%. Die Reaktion ist schematisch in Fig. 5 dargestellt.

### 6. Synthese von MAD-X3

0,3 g (1,2 mmol) DNBTO wurden in 50 ml Ethanol gelöst. Der Lösung wurden 0,25 g (2,4 mmol) Triaminoguanidin zugesetzt. Die Mischung wurde für 30 min bei Raumtemperatur gerührt. Das dabei gebildete Präzipitat wurde als oranger Feststoff durch Filtration abgesondert. Der abgesonderte Feststoff wurde aus Wasser rekristallisiert. Dabei wurden 0,47 g (1,0 mmol) MAD-X3 erhalten. Dies entspricht einer Ausbeute von 83%. Die Reaktion ist schematisch in Fig. 6 dargestellt.

Bei den Synthesen von MAD-X1, MAD-X2 und MAD-X3 ist es vorteilhaft, dass DNBTO eine sehr gute und MAD-X2, MAD-X1 und MAD-X3 eine sehr schlechte Löslichkeit in Ethanol aufweisen. Dadurch können die Verbindungen in guter Ausbeute und mit hoher Reinheit isoliert werden.

Das erfindungsgemäße Verfahren erlaubt eine Synthese aus sehr kostengünstigen, leicht verfügbaren Ausgangsstoffen, Lösungsmitteln und Reaktanten. Das Syntheseverfahren kommt mit einer geringen Zahl an Reaktionsschritten aus und liefert hohe Ausbeuten. Das Verfahren ist damit für einen industriellen Herstellungsprozess gut geeignet. Ein weiterer großer Vorteil besteht darin, dass die im gesamten Verfahren entstehenden Reaktionsprodukte insensitiv gegenüber äußeren Stimuli, wie Reibung und Stoß, sind. Das Verfahren ist dadurch sehr sicher zu handhaben.

## Patentansprüche

1. 3,3'-Dinitro-5,5'-Bistriazol-1,1'-diol.

2. Salz von 3,3'-Dinitro-5,5'-Bistriazol-1,1'-diol.

3. Salz nach Anspruch 2, wobei das Salz ein Dihydroxylammoniumsalz, Diguanidiniumsalz oder Ditriaminoguanidiniumsalz von 3,3'-Dinitro-5,5'-Bistriazol-1,1'-diol ist.

4. Energetische Wirkmasse umfassend ein Dihydroxylammoniumsalz, Diguanidiniumsalz oder Ditriaminoguanidiniumsalz von 3,3'-Dinitro-5,5'-Bistriazol-1,1'-diol.

5. Verwendung eines Dihydroxylammoniumsalzes, Diguanidiniumsalzes oder Ditriaminoguanidiniumsalzes von 3,3'-Dinitro-5,5'-Bistriazol-1,1'-diol als Sprengstoff, Treibmittel, pyrotechnisches Gaserzeugungsmittel oder Additiv zu einem Feststofftreibstoff.

6. Verfahren zur Herstellung von 3,3'-Dinitro-5,5'-Bistriazol-1,1'-diol, wobei 3,3'-Dinitro-5,5'-bis(1*H*-1,2,4-triazol) (DNBT) zum 3,3'-Dinitro-5,5'-bis(1,2,4-triazol)-1,1'-diol (DNBTO) oxidiert wird.

7. Verfahren nach Anspruch 6, wobei das DNBT mittels 2KHSO₅·KHSO₄·K₂SO₄ oder einer anderen anorganischen oder organischen Peroxosäure, Perborat, Wasserstoffperoxid oder hypofluoriger Säure oder einem anderen Sauerstoffüberträger oxidiert wird.

8. Verfahren nach Anspruch 6 oder 7, wobei das DNBT durch Diazotierung von 3,3'-Diamino-5,5'-bis(1*H*-1,2,4-triazol) (DABT) in Schwefelsäure in Gegenwart von Nitrit erhalten wird.

9. Verfahren nach Anspruch 8, wobei das DABT durch Reaktion von Oxalsäure und Aminoguanidinbicarbonat in Säure, insbesondere konzentrierter Salzsäure, Absondern des dabei gebildeten Zwischenprodukts und Erhitzen des Zwischenprodukts in einem basischen Medium erhalten wird.

10. Verfahren zur Herstellung von Dihydroxylammoniumsalz, Diguanidiniumsalz oder Ditriaminoguanidiniumsalz von 3,3'-Dinitro-5,5'-Bistriazol-1,1'-diol mit folgenden Schritten:
a) Inkubation von DNBTO mit Hydroxylamin, Hydroxylammoniumionen, Guanidiniumcarbonat, Guanidiniumionen, Guanidin oder Triaminoguanidin in alkoholischer Lösung und
b) Absondern des dabei erhaltenen Präzipitats.

## Claims

1. 3,3'-dinitro-5,5'-bis-triazole-1,1'-diol.

2. A salt of 3,3'-dinitro-5,5'-bis-triazole-1,1'-diol.

3. A salt as defined in claim 2, which is a dihydroxylammonium salt, a diguanidinium salt, or a di-triaminoguanidinium salt of 3,3'-dinitro-5,5'-bis-triazole-1,1'-diol.

4. An energetic active mass comprising a dihydroxylammonium salt, a diguanidinium salt, or a di-triaminoguanidinium salt of 3,3'-dinitro-5,5'-bis-triazole-1,1'-diol.

5. The use of a dihydroxylammonium salt, a diguanidinium salt, or a di-triaminoguanidinium salt of 3,3'-dinitro-5,5'-bis-triazole-1,1'-diol as an explosive, a propellant, a pyrotechnic gas-producing means, or as an additive to a solid propellant.

6. A method for the production of 3,3'-dinitro-5,5'-bis-triazole-1,1'-diol, wherein 3,3'-dinitro-5,5'-bis-(1*H*-1,2,4-triazole) (DNBT) is oxidized to the 3,3'-dinitro-5,5'-bis-(1,2,4-triazole)-1,1'-diol (DNBTO).

7. A method as defined in claim 6, wherein said DNBT is oxidized by means of 2KHSO₅·KHSO₄·K₂SO₄ or some other inorganic or organic peroxoacid, perborate, hydrogen peroxide, or hypofluorous acid or some other oxygen transfer agent.

8. A method as defined in claim 6 or claim 7, wherein said DNBT is obtained by diazotization of 3,3'-diamino-5,5-bis-(1*H*-1,2,4-triazole) (DABT) in sulfuric acid in the presence of nitrite.

9. A method as defined in claim 8, in which said DABT is obtained by reaction of oxalic acid and aminoguanidine bicarbonate in an acid, more particularly in concentrated hydrochloric acid, separation of the intermediate thus formed, and heating said intermediate in a basic medium.

10. A method for the production of dihydroxylammonium salt, diguanidinium salt, or di-triaminoguanidinium salt of 3,3'-dinitro-5,5'-bis-triazole-1,1'-diol comprising the following steps:
a) incubation of DNBTO with hydroxylamine, hydroxylammonium ions, guanidinium carbonate, guanidinium ions, guanidine or triaminoguanidine in alcoholic solution and
b) separation of the precipitate thus formed.

## Revendications

1. 3,3'-Dinitro-5,5'-bistriazol-1,1'-diol.

2. Sel de 3,3'-dinitro-5,5'-bistriazol-1,1'-diol.

3. Sel selon la revendication 2, dans lequel le sel est un sel de dihydroxylammonium, sel de diguanidinium ou sel de ditriaminoguanidinium de 3,3'-dinitro-5,5'-bistriazol-1,1'-diol.

4. Masse active énergétique comprenant un sel de dihydroxylammonium, sel de diguanidinium ou sel de ditriaminoguanidinium de 3,3'-dinitro-5,5'-bistriazol-1,1'-diol.

5. Utilisation d'un sel de dihydroxylammonium, sel de diguanidinium ou sel de ditriaminoguanidinium de 3,3'-dinitro-5,5'-bistriazol-1,1'-diol comme explosif, propulseur, moyen de génération gazeuse pyrotechnique ou additif à un combustible solide.

6. Procédé de fabrication de 3,3'-dinitro-5,5'-bistriazol-1,1'-diol, dans lequel du 3,3'-dinitro-5,5'-bis(1*H*-1,2,4-triazole) (DNBT) est oxydé en 3,3'-dinitro-5,5'-bis(1,2,4-triazol)-1,1'-diol (DNBTO).

7. Procédé selon la revendication 6, dans lequel le DNBT est oxydé au moyen de 2KHSO₅·KHSO₄·K₂SO₄ ou d'un autre acide peroxydique inorganique ou organique, de perborate, de peroxyde d'hydrogène ou d'acide hypofluoré ou d'un autre vecteur d'oxygène.

8. Procédé selon la revendication 6 ou 7, dans lequel le DNBT est obtenu par diazotation de 3,3'-diamino-5,5'-bis(1*H*-1,2,4-triazole) (DABT) dans de l'acide sulfurique en présence de nitrite.

9. Procédé selon la revendication 8, dans lequel le DABT est obtenu par réaction d'acide oxalique et de bicarbonate d'aminoguanidine dans de l'acide, notamment de l'acide chlorhydrique concentré, séparation du produit intermédiaire formé ce faisant et réchauffement du produit intermédiaire dans un milieu basique.

10. Procédé de fabrication de sel de dihydroxylammonium, sel de diguanidinium ou sel de ditriaminoguanidinium de 3,3'-dinitro-5,5'-bistriazol-1,1'-diol avec les étapes suivantes :
a) incubation de DNBTO avec de l'hydroxylamine, des ions hydroxylammonium, du carbonate de guanidinium, des ions guanidinium, de la guanidine ou de la triaminoguanidine en solution alcoolique et
b) séparation du précipité obtenu ce faisant.
